(19) Europäisches Patentamt
European Patent Office
Office européen des brevets

(11) EP 4 520 836 A1

# (12) EUROPEAN PATENT APPLICATION

(43) Date of publication:
**12.03.2025 Bulletin 2025/11**

(21) Application number: **23306473.2**

(22) Date of filing: **05.09.2023**

(51) International Patent Classification (IPC):
***C12P 7/04*** *(2006.01)* ***C12N 9/18*** *(2006.01)*
***C12N 9/24*** *(2006.01)* ***C12P 7/26*** *(2006.01)*
***C12P 7/62*** *(2022.01)*

(52) Cooperative Patent Classification (CPC):
**C12P 7/26; C12N 9/18; C12N 9/2402; C12P 7/04; C12P 7/62; C12Y 301/01001; C12Y 301/01003; C12Y 301/01013; C12Y 301/01074; C12Y 302/01;** C12R 2001/69; C12R 2001/72

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Essilor International**
**94220 Charenton-le-Pont (FR)**

(72) Inventors:
- **ZAPARUCHA, Anne**
**91440 Bures-Sur-Yvette (FR)**
- **JADHAV, Dipesh**
**31400 Toulouse (FR)**

(74) Representative: **Santarelli**
**Tour Trinity**
**1 bis Esplanade de la Défense**
**92035 Paris La Défense Cedex (FR)**

Remarks:
The complete document including Reference Table(s) and the Sequence Listing(s) can be downloaded from the EPO website

## (54) METHOD AND KIT FOR THE ENZYMATIC DEGRADATION OF POLY(ALLYL DIGLYCOL CARBONATE)

(57) The present disclosure relates to a method for the enzymatic degradation of poly(allyl diglycol carbonate) such as CR-39® polymer, consisting in contacting at least one enzyme of the invention with poly(allyl diglycol carbonate) such as CR-39® polymer in a suitable form, for a sufficient time and in non-toxic and environmentally friendly reaction conditions.

The method can also be used to recover the basic monomer and/or other compounds derived from the enzymatic degradation process, which can be further used to re-obtain the polymer or as intermediates in the synthesis of other chemicals.

The present disclosure also concerns a kit for the enzymatic degradation of poly(allyl diglycol carbonate) such as CR-39® polymer.

EP 4 520 836 A1

## Description

### FIELD OF THE INVENTION

The present Invention belongs to the domain of polymer recycling, and more specifically of polymer recycling via enzymatic degradation.

Indeed, the present Invention concerns a method for the enzymatic degradation of poly(allyl diglycol carbonate), such as CR-39® polymer.

The Invention discloses a method and kit using enzymes capable of degrading poly(allyl diglycol carbonate), such as CR-39® polymer, in non-toxic and environmentally friendly conditions.

By degrading poly(allyl diglycol carbonate) such as CR-39® polymer, the method of the Invention allows reducing polymer waste as well as recovering the basic monomer and/or other chemicals, which can be further used to re-obtain poly(allyl diglycol carbonate) such as CR-39® polymer or as source materials or intermediates in the synthesis of other chemicals.

### BACKGROUND OF THE INVENTION

Plastics are synthetic polymers that are ubiquitous ever since their production began from the year 1950's. Starting from this time, plastics have been produced at an increased rate to meet the demand of the entire world.

Environmental pollution by plastic waste started to be reported already in the 1970s and, by now, accumulation of plastic wastes in the environment is a major global problem because these materials are recalcitrant to natural biodegradation processes.

As a response to this problem, increasing efforts to reduce the plastic waste by disposing off plastics through segregated collection and recycling are underway. Despite these efforts, however, of all the plastic that is produced worldwide, only a minor fraction is at present successfully tackled with. The rest goes into the environment, either disposed of by landfilling, incinerated or in the oceans leading to water pollution. All these waste disposal strategies contribute to environmental pollution.

Even if plastics in the environment are degraded by photo-, bio-, and thermo-oxidative depolymerization as well as friction, such biodegradation can take long periods of time, ranging from 50 years to more than 100 years.

As a consequence, scientists started to investigate ways of dealing with the vast accumulation of plastics that are more efficient and faster with respect to the above mentioned strategies of plastic waste disposal.

It has been found that a number of microorganisms are capable of producing enzymes that can degrade certain polymers (Tsushima *et al.,* 2010). The enzymes that are known to degrade plastic polymers belong to the class "hydrolases". Enzymes belonging to this class are involved in a catalytic reaction which causes the breakdown of chemical bonds of their substrates in the presence of water. The hydrolases capable of degrading certain plastics are capable of hydrolyzing one or more chemical bonds within many of the commonly used plastics. The enzymes so far identified include esterases, lipases, PETases, laccases, polyurethanases and cutinases. The enzymatic degradation of plastic polymers results in simpler fragments, oligomeric or monomeric units. Microorganisms can further assimilate these smaller units into the microbial cell for further enzymatic degradation and release of metabolic products such as $CO_2$, $H_2O$, $CH_4$, and $N_2$ (Amobonye *et al.,* 2021). Many of these enzymes have been isolated from algae, actinomycetes, bacteria and fungi (Urbanek *et al.,* 2020).

Extraction of these enzymes and their modification in order to increase their enzymatic activity has been one of the main research areas in order to cope with ever increasing plastic pollution. Many types of plastics including polyethylene (PE), polyethylene terephthalate (PET), polylactic acid (PLA), polybutylene succinate (PBS) and polyurethane (PU) have been reported to undergo slow degradation in presence of these enzymes (Mukai *et al.,* 1993).

The use of such enzymes not only allows reducing the problem of increasing plastic pollution. In fact, the metabolic by-products of plastic degradation, some of which certainly harmful, can be recovered and re-cycled to re-obtain the original polymers and/or to synthesize other chemicals. This circular economy model allows reducing the environmental impact of plastics, thereby increasing the sustainability of their production.

Poly(allyl diglycol carbonate), such as CR-39® polymer, is a polymer used in a number of applications and, in particular, in the production of ophthalmic lenses. Despite its extensive use and its industrial interest, no method has currently been reported for its enzymatic degradation. Such a method would be of great interest because it would allow the re-cycling of the large quantities of poly(allyl diglycol carbonate) such as CR-39® polymer generated by the industry, therefore contributing to reduce the environmental impact of its production and use.

### DISCLOSURE OF THE INVENTION

As described in the experimental part below, the inventors discovered that certain enzymes are capable of

degrading poly(allyl diglycol carbonate) such as CR-39® polymer.

In light of these findings, the present invention aims to remedy to some or all of the above-mentioned problems of the prior art by proposing a method for the enzymatic degradation of poly(allyl diglycol carbonate) such as CR-39® polymer.

It should be noted that, at the time of filing of the present Application, no enzyme and method for the enzymatic degradation of poly(allyl diglycol carbonate) such as CR-39® polymer have been disclosed.

As used herein, the terms "comprise", or variations such as "comprises" or "comprising" as well as "including" and variations such as "include", "includes," and "included", are not limiting.

The term "about", when used before a numerical value, indicates that the value may vary within reasonable range, such as ± 10%, ± 5%, and ± 1%. The expression "about x" includes the value "x."

As used herein, the term "polymer" refers to a chemical compound which comprises repeating units linked by covalent chemical bonds.

As used herein, the term "repeating unit" is a part of a polymer whose repetition produces the complete polymer chain (except for the end-groups) by linking the repeat units together along the chain.

The repeating unit is not to be confused with the term "monomer", which refers to the small molecule from which a polymer is synthesized. In this sense, for example, repeating units can also be made from two or more monomers.

As used herein, poly(allyl diglycol carbonate) is a polymer characterized in that the repeating unit is allyl diglycol carbonate monomer also known as 2-(2-prop-2-enoxycarbonyloxyethoxy)ethyl prop-2-enyl carbonate and diethyleneglycol bis allylcarbonate. The formula of the monomer from which the repeating units of the poly(allyl diglycol carbonate) are derived is the following:

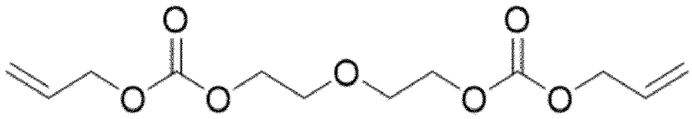

Alternative names of CR-39® polymer include: 2,5,8,10-Tetraoxatridec-12-enoic acid, 9-oxo-, 2-propen-1-yl ester, homopolymer. The CAS number of this polymer is 25656-90-0. The formula thereof is:

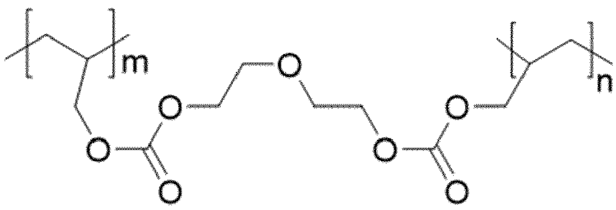

CR-39® is a registered trademark of PPG. Other suppliers of poly(allyl diglycol carbonate) are known. For example, Chunling is supplying such polymer under the trade name TOM 1000.

As used herein, the term "oligomer" refers to the polymer of the invention consisting of 2 to 50, preferably 2 to 20 repeating units.

The polymer according to the invention identifies an article comprising or deriving from domestic, commercial or industrial mixed or sorted waste streams or collected plastic wastes and it can be characterized by any physical conformation, e.g. sheets, films, tubes, pipes, straws, rods, ropes, strings, lines, nets, reticulated sheets, three dimensional regular shapes, blocks, sheaths, fibers, membranes, woven and non-woven textiles, bags, containers, bottles, capsules, packaging, microspheres, (electro)mechanical components, clothing, coatings and panels.

As used herein, the terms "enzymatic degradation", "polymer degradation", "depolymerization" and "enzymatic hydrolysis" are used interchangeably and they refer to a chemical process wherein a polymeric material undergoes degradation by one or more degradative enzymes, which cleave one or more chemical bonds between and/or inside the repeating units of the polymer. The result of the enzymatic degradation is degradation products, which are the repeating units, monomers, oligomers and/or fragments of the polymer.

The terms "complete degradation" and "complete depolymerization" mean that the polymer according to the invention is entirely converted into the repeating unit(s) of which it is composed. On the contrary, the terms "partial degradation" and "partial depolymerization" mean that the polymer according to the invention is only partly converted into the repeating unit(s) of which it is composed, *i.e.* a part of the polymer is converted into oligomers and/or fragments.

As used herein, the term "degradative enzyme" refers to an enzyme capable of degrading poly(allyl diglycol carbonate) such as CR-39® polymer.

According to a first embodiment, the invention concerns a method for the enzymatic degradation of poly(allyl

diglycol carbonate) comprising contacting said polymer with at least one enzyme capable of degrading poly(allyl diglycol carbonate) and selected from the group consisting of:

- enzyme 1, the amino acid sequence of which presents at least 60% identity with the following amino acid sequence:

MKLLSLTGVAGVLATCVAATPLVKRLPSGSDPAFSQPKSVLDAGLTCQGASP
SSVSKPILLVPGTGTTGPQSFDSNWIPLSTQLGYTPCWISPPPFMLNDTQVNTEYMV
NAITALYAGSGNNKLPVLTWSQGGLVAQWGLTFFPSIRSKVDRLMAFAPDYKGTVL
AGPLDALAVSAPSVWQQTTGSALTTALRNAGGLTQIVPTTNLYSATDEIVQPQVSNS
PLDSSYLFNGKNVQAQAVCGPLFVIDHAGSLTSQFSYVVGRSALRSTTGQARSADY
GITDCNPLPANDLTPEQKVAAAALLAPAAAAIVAGPKQNCEPDLMPYARPFAVGKRT
CSGIVTP (SEQ ID NO: 1 in the annexed sequence listing),

- enzyme 2, the amino acid sequence of which presents at least 60% identity with the following amino acid sequence:

MWLLPLVLTSLASSATWAGQPASPPVVDTAQGRVLGKYVSLEGLAQPVAVF
LGVPFAKPPLGSLRFAPPQPAEPWSFVKNTTSYPPMCCQDPVVEQMTSDLFTNGK
ERLTLEFSEDCLYLNIYTPADLTKRGRLPVMVWIHGGGLVLGGAPMYDGVVLAAHE
NVVVVAIQYRLGIWGFFSTGDEHSRGNWGHLDQVAALHWVQENIANFGGDPGSVT
IFGESAGGESVSVLVLSPLAKNLFHAISESGVALTVALVRKDMKAAAKQIAVLAGCKT
TTSAVFVHCLRQKSEDELLDLTLKMKFLTLDFHGDQRESHPFLPTVVDGVLLPKMPE
EILAEKDFNTVPYIVGINKQEFGWLLPTMMGFPLSEGKLDQKTATSLLWKSYPIANIP
EELTPVATDKYLGGTDDPVKKKDLFLDLMGDVVFGVPSVTVARQHRDAGAPTYMY
EFQYRPSFSSDKKPKTVIGDHGDEIFSVFGFPLLKGDAPEEEVSLSKTVMKFWANFA
RSGNPNGEGLPHWPMYDQEEGYLQIGVNTQAAKRLKGEEVAFWNDLLSKEAAKK
PPKIKHAEL (SEQ ID NO: 2 in the annexed sequence listing),

- enzyme 3, the amino acid sequence of which presents at least 60% identity with the following amino acid sequence:

MDLRTMTQSLVTLAEDNMAFFSGQGPGETARRLSGVFAGIREQALGLEPAL
GRLLSVAHLFDLDAETPANGYRSLVHTARCCLAHLLHKSRYVASNRRSIFFRTSHNL
AELEAYLAALTQLRALAYYAQRLLAINRPGKLFFEGDEGITADFLREYVTLHKGCFYG
RCLGFQFTPAIRPFLQTISIGLVSFGEHYKRNETGLSVTASSLFTSGRFAIDPELRGA
EFERIIQNLDVHFWKAFWNITEIEVLSSLANMASATVRVSRLLSLPPKAFEMPLTADP
KLTVTISPPLAHTGPGPVLVRLISYDLREGQDSEELSSLVRSEGPRGLELRPRPQQA

PRSRSLVVHIHGGGFVAQTSKSHEPYLKSWAQELGVPILSIDYSLAPEAPFPRALEE
CFYAYCWAVKHCGLLGSTGERICLAGDSAGGNLCFTVSLRAAAYGVRVPDGIMAAY
PATMLQSAASPSRLLSLMDPLLPLSVLSKCVSAYAGGEMEDHSDSDQKALGMMGL
VRRDTALLFRDLRLGASSWLNSFLELSGHKSRPNLVPTEEPMRRSVSEAALAQPEG
PLGTDSLKYLTLHDLSLSSETQDTPELSLSAETLGPTTPSAVNFLFRPEDAPEEAEA
RDDISTKEEKVYSVRAAFPEGFHPRRSSQGAIQMPLYSAPIVKNPFMSPLLAPDSML
QTLPPVHIVACALDPMLDDSVMFARRLRSLGQPVTLHVVEDLPHGFLSLAALCRETR
QAAALCVDRIRFILNPPGPATPAGPTTPPV (SEQ ID NO: 3 in the annexed sequence listing),

- enzyme 4 corresponding to a lipase from *Aspergillus oryzae,*

- enzyme 5, the amino acid sequence of which presents at least 60% identity with the following amino acid sequence:

MRKFTLGCLSATALVALSVSATAAPLPDTPGAPFPSVSSFDNDGPYAVTSQS
EGPNCRVYRPRTLGQGGVRHPIILWGNGTGTGPTTYSGLLTHWASHGFVVAAAET
SNAGTGREMLACLDYLVQESNRTYGTYVGVLNTGRVGTSGHSQGGGGSIMAGQD
DRVKATAPIQPYTIGLGHDSSSQRNQRGPMFLMSGGADTIAIPYLNAQPVFTRANVP
IFWGERRYVSHFEPVGNGGEYRGPSTAWFRYQLMDDQSARSTFYGRLCRLCTSLL
WSVERKGIE (SEQ ID NO: 4 in the annexed sequence listing),

- enzyme 6, the amino acid sequence of which presents at least 60% identity with the following amino acid sequence:

MAVMTPRRERSSLLSRALQVTAAAATALVTAVSLAAPAHAANPYERGPNPT
DALLEASSGPFSVSEENVSRLSASGFGGGTIYYPRENNTYGAVAISPGYTGTEASIA
WLGERIASHGFVVITIDTITTLDQPDSRAEQLNAALNHMINRASSTVRSRIDSSRLAV
MGHSMGGGGTLRLASQRPDLKAAIPLTPWHLNKNWSSVTVPTLIIGADLDTIAPVAT
HAKPFYNSLPSSISKAYLELDGATHFAPNIPNKIIGKYSVAWLKRFVDNDTRYTQFLC
PGPRDGLFGEVEEYRSTCPF (SEQ ID NO: 5 in the annexed sequence listing),

- enzyme 7, the amino acid sequence of which presents at least 60% identity with the following amino acid sequence:

MTELTVQTRCGALKGTAGHGVRTWKSIPYAKPPVGELRFKAPEPPVPWDGV
KNADSFGPVCPQPADLLSMSFSGDVPPQSEDCLYLNVFAPDSEGGKRPVMVWIHG
GAFFLGAGSEPTYDASALAADGDVIVVTLNYRLGPFGFLHLFSIDDTYPGNIGMLDQI
AALRWVKDNISAFGGDPDNVTVFGESAGGMSIASLMAMPDAKGLFHKAILESGASQ
TMTADVAKEITTAFIQEAGTDQLQELSVNDILKTADKLRNTIDQSIFHLLFQPAIDPATL
PAEPAKAIADGAAEGIPMIIGTNRDEAYLFFTPDTDIHSEKKQQEYLLYHLGENSAKL
AADLYPHSLTGQIDMMTDLKFWRPAVAFAQEQSQYAPVWMYRFDWHGETPPFHK
AVHALELPFVFGNFDSLKKTLKEPLSEDVKQLSKLIQSAWIAFAKTGKPDTDQLHWP

QYETGSRETIIFNTSVSTESDPDSAKRRILFQA (SEQ ID NO: 6 in the annexed sequence listing),

- enzyme 8, the amino acid sequence of which presents at least 60% identity with the following amino acid sequence:

MSASSLKFPIVLVHGLLGFDKIGGIYPYFYGIKEALEKAGAKVYIATLSALNSNE LRGEQLLEFVRKVQAETGAAKVNLIGHSQGPLACRYVAATHPELIASVTSVNGVNH GSEVADLVRLALTPGRLPESIANAAMSAFGQLLSALAGSPRLPQSGIEALEALTSEG VAAFNKKYPQGLPAEWGGEGKELVNGVYYYSWSGIIDYNPLHQGANNLDPLHVAM LAFSILFTNERFQNDGLVGRYSSHLGKVIGSDYSMDHVDAINQLAGVVANNTDPVQL FVEHVARLKSKGL (SEQ ID NO: 7 in the annexed sequence listing),

and

- enzyme 9, the amino acid sequence of which presents at least 60% identity with the following amino acid sequence:

MKKLVALLIVGLFVSISLTGLGLAGGTAPYKCDTRYPVVLAHGMGASTKILGIV DYWYGIEDALKAEGASVYFTSVNAMGSTVDKAADFKQQFMEILAVTGAPKANIIGHS HGTLYTRYAISNLGLAPYVASYTSLAGPHRGSAVASLIMYDLPDWLLAAGGDVLNFV YTFIFGDTNPDSLQNALDLCPDYMVNTFNPNTPNIPGIYYQSWAAKAKTSCPSVILEP TWLIMLIEEGANDGLVSVESAKWGNFRGVEDAAWYSAGCDHLNIVGQLFGVTPGF DAPQFFVDIVEDLKGRGY (SEQ ID NO: 8 in the annexed sequence listing).

On one hand, hydrolytic enzymes or hydrolases capable to cleave/hydrolyse the carbonate function such as lipases, proteases, polyurethanases, cutinases, and esterases and, on the other hand, laccases are designated as degradative enzymes in the sense of the present invention.

The degradative enzymes selected from the group consisting of enzymes 1 to 3 and enzymes 5 to 9 may be of any suitable origin, such as vegetable, animal, bacterial, fungal and yeast origin. Origin can further be mesophilic or extremophilic (psychrophilic, psychrotrophic, thermophilic, barophilic, alkalophilic, acidophilic, halophilic, *etc.*). In general, any microorganism capable of expressing the enzymes disclosed herein, whether naturally or by recombinant DNA techniques and recombinant protein expression methods, may be suitable to carry out the method of the invention. In a particular embodiment, enzyme 7 as previously defined is mesophilic.

The enzymes implemented in the invention can be conveniently produced by recombinant techniques using a cellular expression system. Any suitable expression system may be used, including those involving prokaryotic cells and eukaryotic cells. Suitable prokaryotic cells may be any Gram-positive or Gram-negative bacterium. Gram-positive bacteria include, but are not limited to, *Bacillus, Clostridium, Corynebacterium, Enterococcus, Geobacillus, Lactobacillus, Lactococcus, Oceanobacillus, Staphylococcus, Streptococcus,* and *Streptomyces.* Gram-negative bacteria include, but are not limited to, *Acinetobacter, Campylobacter, E. coli, Flavobacterium, Fusobacterium, Helicobacter, Ilyobacter, Neisseria, Pseudomonas, Salmonella,* and *Ureaplasma.* Particularly preferred as a heterologous host are *E. coli* strains engineered to export the enzymes to the cell surface or extracellularly. Suitable eukaryotic cells may be selected from: yeasts, fungi, insect cells, mammalian cells, and plant cells. Suitable yeast cells may be selected from: *Candida, Hansenula, Kluyveromyces, Pichia, Saccharomyces* such as *Saccharomyces cerevisiae, Schizosaccharomyces* such as *Schizosaccharomyces pombe,* or *Yarrowia* cell. Suitable fungal cells may be selected from a filamentous fungus, for example *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* and *Trichoderma.*

As used herein, the term "recombinant" refers to any type of material that has been produced by any of the techniques of recombinant DNA technology, for example using recombinant nucleic acids, Polymerase Chain Reaction (PCR), *etc.* Recombinant proteins are proteins produced by the expression of recombinant nucleic acids and the enzymes disclosed in the present Application can be recombinant enzymes.

The enzymes implemented in the invention can equally be produced using other techniques known in the art, including direct modifications of isolated enzymes and direct protein synthesis.

Alternatively, the enzymes implemented in the invention can also be commercially available and used according to the manufacturer's instructions.

As used herein, the term "sequence identity" or "identity" in relation to an amino acid sequence defines the number (or fraction when expressed as a percentage, %) of identical amino acid residues between two polypeptide sequences. The sequence identity is determined by comparing the sequences when aligned so as to maximize overlap and identity while minimizing sequence gaps, and may be determined using any of a number of alignment algorithms known to those skilled in the art. In particular, sequence identity may be determined using computer programs (such as the BLASTP program publicly available from NCBI and other sources) or direct sequence comparison.

According to a specific aspect, the amino acid sequences of the enzymes implemented in the invention is at least about 65%, 70%, 75%, 80%, 85%, 90%, 95%, 96%, 97%, 98%, 99% or even 100% identical to the amino acid sequences set forth in the sequences provided herein and possess the same enzymatic function.

In other terms, such enzyme variants retain the enzymatic activity of the enzymes whose sequence is set forth in the sequences provided herein, and include mutants differing by the addition, deletion or substitution of one or more amino acid residues. The variant may have one or more conservative changes, wherein a substituted amino acid has similar structural or chemical properties (e.g., replacement of leucine with isoleucine). Alterations may occur at the amino- or carboxy-terminal positions of the reference polypeptide sequence or anywhere between those terminal positions, interspersed either individually among the amino acids in the reference sequence or in one or more contiguous groups within the reference sequence.

In a particular embodiment, the amino acid sequence of enzyme 1 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 1.

Advantageously, the amino acid sequence of enzyme 1 used in the method according to the invention comprises or consists of SEQ ID NO: 1.

In a more particular embodiment, the enzyme 1 as implemented in the invention is a lipase B, preferably a lipase B from *Candida antarctica* and more preferably a commercial lipase B from *Candida antarctica*

In an even more particular embodiment, the enzyme 1 as implemented in the invention is the commercial lipase Lipozyme® CAL-B. In other words, the enzyme 1 implemented in the present invention is commercially available under the name Lipozyme® CAL-B, which is produced and supplied by, for example, Codexis, Novozymes A/S or Strem Chemicals, Inc.

As used herein, the term "commercial" and the expression "commercially available" refer to an enzyme that is produced by a company and that can be purchased from said company in an essentially pure form and with a given activity, measured in U, where U is the unit of enzyme's catalytic activity, and 1U is defined as the amount of the enzyme that catalyzes the conversion of one micromole of substrate per minute under the specified conditions of the assay method.

In a preferred embodiment, enzyme 1 as defined according to any one of the above embodiments is used in the method of the invention under the form of a crude lysate.

In a particular embodiment, the amino acid sequence of enzyme 2 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 2.

Advantageously, the amino acid sequence of enzyme 2 used in the method according to the invention comprises or consists of SEQ ID NO: 2.

In a more particular embodiment, the enzyme 2 as implemented in the invention is an esterase, preferably an esterase from porcine liver and more preferably an esterase from porcine liver the CAS Number of which is 9016-18-6.

In an even more particular embodiment, the enzyme 2 as implemented in the invention is a commercial esterase from porcine liver. For example, the enzyme 2 implemented in the present invention is the esterase from porcine liver commercially available under the product number "E3019", which is produced and supplied by Sigma-Aldrich (Merck).

In a particular embodiment, the amino acid sequence of enzyme 3 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 3.

Advantageously, the amino acid sequence of enzyme 3 used in the method according to the invention comprises or consists of SEQ ID NO: 3.

In a more particular embodiment, the enzyme 3 as implemented in the invention is a cholesterol esterase, preferably a cholesterol esterase from porcine pancreas and more preferably a cholesterol esterase from porcine pancreas the CAS Number of which is 9026-00-0.

In an even more particular embodiment, the enzyme 3 as implemented in the invention is a commercial cholesterol esterase from porcine pancreas. For example, the enzyme 3 implemented in the present invention is the cholesterol esterase from porcine pancreas commercially available under the product number "26745", which is produced

and supplied by Sigma-Aldrich (Merck).

In a preferred embodiment, enzyme 3 as defined according to any one of the above embodiments is used in the method of the invention under the form of a crude lysate.

In a particular embodiment, the enzyme 4 as implemented in the invention is the commercial lipase Novozym® 51032 from *Aspergillus oryzae.* In other words, the enzyme 4 implemented in the present invention is commercially available under the name Novozym® 51032, which is produced and supplied by, for example, Novozymes A/S or Strem Chemicals, Inc.

In a particular embodiment, the amino acid sequence of enzyme 5 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 4.

Advantageously, the amino acid sequence of enzyme 5 used in the method according to the invention comprises or consists of SEQ ID NO: 4.

According to an even more particular embodiment, enzyme 5 used in the method according to the invention belongs to the enzyme family of lipases.

In a particular embodiment, the amino acid sequence of enzyme 6 as implemented in the invention exhibits at least 60% identity and can exhibit at least 65%, 70%, 75%, 80%, 85%, 90%, 95%, 96%, 97%, 98%, 99% or 100% identity with the amino acid sequence SEQ ID NO: 5.

Advantageously, the amino acid sequence of enzyme 6 used in the method according to the invention comprises or consists of SEQ ID NO: 5.

According to an even more particular embodiment, enzyme 6 used in the method according to the invention belongs to the enzyme family of cutinases.

In a preferred embodiment, enzyme 6 as defined according to any one of the above embodiments is used in the method of the invention under the form of a crude lysate.

In a particular embodiment, the amino acid sequence of enzyme 7 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 6.

Advantageously, the amino acid sequence of enzyme 7 used in the method according to the invention comprises or consists of SEQ ID NO: 6.

According to an even more particular embodiment, enzyme 7 used in the method according to the invention belongs to the enzyme family of carboxylesterases/lipases.

In a preferred embodiment, enzyme 7 as defined according to any of the above embodiments is used in the method of the invention under the form of a purified protein.

In a particular embodiment, the amino acid sequence of enzyme 8 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 7.

Advantageously, the amino acid sequence of enzyme 8 used in the method according to the invention comprises or consists of SEQ ID NO: 7.

According to an even more particular embodiment, enzyme 8 used in the method according to the invention belongs to the enzyme family of putative lactonizing lipases.

In a preferred embodiment, enzyme 8 as defined according to any one of the above embodiments is used in the method of the invention under the form of a crude lysate or of a purified protein.

In a particular embodiment, the amino acid sequence of enzyme 9 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 8.

Advantageously, the amino acid sequence of enzyme 9 used in the method according to the invention comprises or consists of SEQ ID NO: 8.

According to an even more particular embodiment, enzyme 9 used in the method according to the invention belongs to the alpha/beta hydrolase superfamily.

According to the invention, two or more enzymes as above defined (i.e. enzymes 1-9) can be used at the same time for enzymatic degradation of poly(allyl diglycol carbonate) such as CR-39® polymer. The two or more enzymes can interact in the reaction mixture and the result of their interaction can be additive (i.e., the degradative activity of the enzymes is the sum of the activities of the enzymes if they were acting separately) or synergistic (*i.e.*, the degradative activity of the enzymes is more than the sum of the activities of the enzymes if they were acting separately). When three or more enzymes as above defined (*i.e.* enzymes 1-9) are present, both additive and synergistic effects can be at play at the same time. Other enzyme(s) can also be present in the reaction mixture, in addition to the enzyme(s) disclosed in the present invention.

Before enzymatic degradation according to the method of the invention, poly(allyl diglycol carbonate) such as CR-39® polymer can be submitted to a pre-processing step aiming at promoting or accelerating the enzymatic degradation

by altering the physical state of the polymer. This pre-processing step physically changes the structure of the polymer, for example by increasing the surface area available for contact with the degradative enzyme.

Pre-processing is preferably conducted before, or during, contact with the enzyme(s) as implemented in the invention, *i.e.* as a pre-treatment or as a co-processing step. Preferably, pre-processing is conducted as a pre-treatment processing step.

Pre-processing may, for example, comprise mechanical processing of the polymer by a treatment selected from: washing, centrifugation, cleaning, collision, sorting, grinding, homogenization, shredding, cutting, impacting, crushing, shearing, shredding, rotary drum tumbling, fractionation, sonication, melting, extrusion, spinning, liquefaction, maceration, micronization, pelletization, screw pressing, piston pressing and granulation, or a combination of two or more of the preceding methods. Grinding is particularly preferred, optionally together with one or more of the other pre-processing steps listed above.

Alternatively, or in addition, poly(allyl diglycol carbonate) such as CR-39® polymer may be physically processed by a treatment selected from: irradiation, for example drying, UV irradiation, amorphization, agglomeration, heating (e.g. by microwaves), cooling, freezing, dessication, or a combination of two or more of the preceding methods.

In the method according to the Invention, poly(allyl diglycol carbonate) such as CR-39® polymer can be put in contact with at least one enzyme as above defined in any appropriate way, *i.e.* in any way that allows the enzyme(s) to interact with poly(allyl diglycol carbonate) such as CR-39® polymer so that it can exert its degrading activity. Non-limiting examples of contacting the enzyme(s) and polymer are the followings:

- poly(allyl diglycol carbonate) such as CR-39® polymer can be dispersed in the solution containing the enzyme(s). In this case, the polymer can be in any form, e.g., it can be added as a film, sheet or as a granular material. Other polymer forms, e.g. mouldings, can be added as an integral whole or in comminuted form. Coated or adhesively bonded materials, or materials on which coatings of the polymer have been deposited, such as paper or cardboard as well as coated paper or coated cardboard, can be added as an integral whole or in comminuted form to the enzyme-containing solution;

- the enzyme solution can be sprayed onto poly(allyl diglycol carbonate) such as CR-39® polymer: the aqueous enzyme-containing solution can be sprayed or deposited by spraying onto the polymer to be degraded. In this case, the enzyme solution should completely cover the polymer, whatever its form;

- poly(allyl diglycol carbonate) such as CR-39® polymer may be contacted directly with the enzyme-expressing organism. Unpurified (cell lysates) or semipurified culture supernatants containing the enzyme(s) may also be contacted with the polymer.

Hence, poly(allyl diglycol carbonate) such as CR-39® polymer may be present in bulk or as a solution, preferably as an aqueous solution or water. The aqueous solution which is required for carrying out the method according to the invention can be buffered. The pH and temperature depend on the specific enzyme(s) used, as the activity of each enzyme is optimal at different pH and temperature values.

The pH is generally between 2 and 12, preferably between 5 and 9 and most preferably between 6 and 8. The temperature at which enzymatic degradation is carried out is generally between 5 and 60°C; it is preferably between 20 and 50°C, and most preferably between 30 and 50°C. Non aqueous solvents/water mixtures can also be used as solvents. Non aqueous solvents can be organic solvents, deep eutectic solvents or natural deep eutectic solvents.

The following are examples of buffers which can be used according to the invention: citrate, acetate, phosphate, formate, carbonate, tris-hydroxymethylaminomethate, triethanolamine, imidazole, oxalate, tartrate, fumarate, maleate, phthalate, succinate and ethylenediamine, as well as a plurality thereof. Acetates, phosphates and tris-hydroxymethylaminomethates are preferably used as buffers.

Poly(allyl diglycol carbonate) such as CR-39® polymer may be contacted with the enzyme(s) as above-defined at a concentration and temperature for a time sufficient to achieve the desired amount of degradation. Suitable times range from a few hours to several days and may be selected to achieve a desired amount of conversion. Examples of reaction times include 1 day, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 days. In some embodiments, reaction times may be one or more weeks. Those skilled in the art will be able to determine an appropriate reaction time by reference to the physical state of the polymer, the enzyme(s) selected and the extent of degradation required.

The method can comprise agitating the reactants to improve contact between the enzyme(s) and the polymer and so promote adsorption of the enzyme(s) onto the poly(allyl diglycol carbonate) such as CR-39® polymer. This step may comprise continuous stirring, for example at a rate of between 100 revolutions per minute (rpm) and 5000 rpm.

Chemical elements and/or compounds that may be necessary for the enzymatic reaction or that may have a positive influence on it, can also be added to the reaction mixture. Preferably, the chemical elements and/or compounds

are selected and added to the reaction mixture for their positive impact on enzyme activity. Examples of chemical elements and/or compounds with positive impact on enzyme activity include, but are not limited to, metal ions (e.g., sodium or calcium ions) and salts (added, e.g., to regulate the ionic strength of the solution).

The whole process can be carried out in any appropriate container, for example a large reactor vessel comprising a mixer and surrounded by a heating mantle to regulate the temperature.

The concentration of the enzyme(s) as implemented in the invention should suffice to quantitatively degrade the polymer. This degradation may be partial or complete. The amount of enzyme(s) required to carry out the method of the invention can be readily determined by the skilled person by reference, *inter alia,* to the relative amount of the polymer and/or the number of any additional enzyme present in the reaction mixture.

For example, in preferred embodiments, the enzyme(s) as implemented in the invention is used in an amount up to 5% by weight of poly(allyl diglycol carbonate) such as CR-39® polymer, more preferably up to 1%, even more preferably up to 0.1%, and yet more preferably up to 0.05% by weight of the polymer. For example, the amount of enzyme of the invention may be in a range of 0.001% to 5% by weight of the polymer, preferably in the range of 0.002% to 1%, more preferably in the range of 0.003% to 0.1%, and even more preferably in the range of 0.005% to 0.05% by weight of the polymer.

According to another embodiment of the method according to the present invention, the at least one degradative enzyme as previously defined is used in the form of a pure protein, a crude lysate or immobilized on a support. Preferably, the degradative enzyme as previously defined is used in the form of a pure protein or of a crude lysate.

Herein, "in pure form" is used to mean in "substantially pure form", *i.e.,* a purity that allows for the effective use of the protein in the method described herein. For a protein to be most useful in the method of the invention or in any method utilizing enzymes described herein, it is most often substantially free of contaminants, other proteins and/or chemicals that might interfere or that would interfere with its use in the method (e.g., that might interfere with enzyme activity).This form can also be known as "cell-free lysate" which is obtained after centrifuging and filtering the lysate obtained during classical protein over-expression.

As used herein, the expression "substantially pure form" means that the enzyme should be at least 90% pure, preferably 95% pure and even more preferably 98% pure.

As used herein, the expressions "crude lysate" or "cell lysate" are equivalent and can be used interchangeably. Both expressions refer to a specific product obtained during classical protein over-expression.

In particular, a crude lysate is what is obtained when the host cells (e.g., *Escherichia coli* cells), after they have been allowed to over-express the protein of interest in culture media, are lysed with any of the methods known in the art, with the aim to liberate in the supernatant the soluble over-expressed protein that is present in the cell.

Different methods can be used depending on the type of host cells and on the quantity of host cells to be lysed. Examples of methods for cell lysis comprise liquid shear pressure techniques (e.g., French press and homogenizer), ultrasonication, freeze and thawing (coupled with enzymatic lysis, e.g. with lysozyme), glass beads, osmotic shock and chemical lysis (e.g., by using detergents).

Despite containing the protein in a non-pure form, because all cell debris and chemicals are still present, in certain cases cell lysates can effectively be used in place of the enzymes in their pure form. This can be the case when, for example, the concentration of the enzyme is particularly high. The main advantage in the use of cell lysates is that this avoids further steps of protein purification, which requires additional time and which increases the costs of the degradation process.

According to the invention, the enzyme(s) may be present in the composition in an immobilized form. Immobilization is often implemented to improve the stability of the enzyme(s). In this case, the enzyme has been immobilized on a suitable support by means of any immobilization method. For example, the enzyme can be immobilized onto solid supports, matrices or particles (for example selected from glass beads and mineral, polymer or metallic particles or matrices).

The immobilization method is based either on physical techniques such as adsorption, entrapment, and encapsulation or on chemical bonding processes such as covalent bonding and crosslinking.

The reaction products of the enzymatic degradation carried out according to the method of the invention comprise one or more compounds selected from the group consisting of diethylene glycol (DEG), a diol compound and $CO_2$. One or more of these compounds can be identified by suitable analytical techniques to evaluate the course of the reaction. Hence, the identification of one or more of such compounds advantageously allows establishing whether the enzymatic degradation has taken place or is taking place.

As shown by the inventors in the hereinafter experimental part, diethylene glycol, the diol compound and $CO_2$ can conveniently be measured, respectively, by Gas chromatography-mass spectrometry (GC-MS), Gel permeation chromatography (GPC) or Fourier-transform infrared spectroscopy (FTIR) and by Bromothymol blue assay (BTB assay). Other techniques known to the skilled person may equally be used.

It will be appreciated that the enzymatic degradation by the enzymes according to the method of the invention need not completely depolymerize the polymer, and that the nature and extent of the degradation may be determined, *inter*

*alia,* by the enzyme selected, the reaction conditions and the chemical nature and/or physical form of the polymer.

More particularly, factors that control the rate at which the polymeric material degrades include the concentration of the enzyme, the enzyme kinetics with respect to polymer degradation, the polymer matrix, the polymer shape (e.g., changing the shape may affect the rate of substrate transport into and out of the material), the environment in which the reaction takes place (e.g., the environment can vary in temperature, pH, ionic strength and other factors that alter enzyme activity) and the fluid dynamics of the outer environment. Other factors that can also alter the rate of polymer degradation are readily available to those with ordinary skills in the art.

From the above, it is thus apparent that the enzymatic degradation mediated by enzymes according to the invention may yield fragments, monomers and/or oligomers of poly(allyl diglycol carbonate) such as CR-39® polymer.

Consequently, the method according to the present invention may further comprise the step of fractionating, isolating or purifying degradation product(s) obtained. Exemplary methods for purification/isolation/separation of degradation products comprise: filtration, distillation, solvent extraction (e.g., liquid/liquid extraction), precipitation, crystallization, evaporative concentration, evaporative crystallization, affinity chromatography, ion exchange chromatography, solvent extraction, centrifugation, electrophoresis, electrodialysis, hydrophobic interaction chromatography, gel filtration chromatography, reverse phase chromatography, differential solubilization, high performance liquid chromatography (HPLC), and/or or reversed-phase HPLC and combinations of two or more of the preceding methods.

The reaction product(s) may also be recovered and re-cycled for obtaining other compounds of interests. For example, these reaction product(s) are allyl alcohol, polyvinyl ethanol, ethylene glycol and diethylene glycol.

Thus the present invention concerns the use of the method as previously defined for producing fragments, oligomers, monomers or repeating units derivatives of poly(allyl diglycol carbonate) such as CR-39® polymer, that can be recovered and re-cycled for obtaining poly(allyl diglycol carbonate) such as CR-39® polymer.

For example, allyl alcohol, ethylene glycol and diethylene glycol can be recovered by and re-cycled for obtaining poly(allyl diglycol carbonate) such as CR-39® polymer.

In addition, allyl alcohol, ethylene glycol and/or diethylene glycol thus recovered may be recycled in any synthesis implementing such products.

According to another embodiment, the invention also concerns a kit for the enzymatic degradation of poly(allyl diglycol carbonate) such as CR-39® polymer, comprising at least one enzyme as previously defined.

According to another embodiment, the kit further comprises at least one reagent for the degradation reaction.

The kit according to the present Invention can optionally include at least one other reagent required to conduct the enzymatic degradation such as buffers, salts, enzyme co-factors and the like. The kit may additionally include one or more controls.

Other components may also be included in the kit. The various components of the kit are optionally provided in suitable containers. The kit can further include containers for holding or storing the polymer. Where appropriate, the kit may also optionally contain reaction vessels, mixing vessels and other components that facilitate the preparation of reagents and/or the polymer.

Other features and advantages of the present invention will become apparent from the following detailed description which makes reference to the accompanying figure.

## FIGURE LEGEND

**Figure 1: Degrading activity of the enzymes of the invention towards CR-39® polymer.** Degrading activity of the 9 enzymes of the invention, determined after contacting a CR-39® polymer cube with each enzyme separately at 50°C for 35days, and by calculating the weight loss of the CR-39® polymer cube at the end of the reaction according to the formula in Example 1.

## DETAILED DESCRIPTION OF PARTICULAR EMBODIMENTS

### Materials & Methods

#### *Bromothymol blue (BTB) high-throughput assay for screening and selection of the enzymes with the highest degrading activity using CR-39® monomer as model substrate*

In order to determine the enzymes with the highest degrading activity, a high-throughput BTB assay was used. This assay makes use of bromothymol blue, which is a pH indicator and therefore changes its color as a function of the pH of the reaction mixture. In the presence of a degradative enzyme, the substrate is degraded in various products, including $CO_2$, which has the effect to decrease the pH of the reaction mixture because of the formation of carbonic acid as indicated in the equation below. This assay thus allows to easily detecting the $CO_2$ released during enzymatic degradation.

$$CO_{2(aq.)} \rightleftharpoons H_2CO_3 \rightleftharpoons HCO_3^{-} + H^{+}$$

Carbonic acid | Bicarbonate ions

As the enzymatic degradation of CR-39® polymer is a slow process, a model substrate similar in structure and functional groups to the CR-39® polymer was used to screen the numerous enzymes in a short time period. The chosen model substrate is CR-39® monomer.

Various parameters of the BTB assay were optimized such as absorption maxima, BTB concentration range, concentration of the buffer to be used *etc.* (Shimada and Hasegawa, 2017). After optimization of these parameters, a bicarbonate calibration curve was obtained (Ocal *et al.,* 2021). This calibration curve obtained with optimized parameters (5 mM, sodium phosphate buffer, pH = 7.0, BTB concentration of 135 μM) was used to evaluate the degrading activity of the enzymes towards CR-39® monomer.

Table 1 lists the 9 enzymes with the highest degrading activity towards CR-39® monomer that were selected using the BTB assay:

**Table 1: Enzymes with highest degrading activity towards allyl diglycol carbonate (CR-39® monomer) as determined by BTB high-throughput assay. 'In-house' means that the enzyme has not been purchased but has been cloned, expressed and purified by the inventors.**

| Enzyme as previously defined | Origin | Details |
|---|---|---|
| Enzyme 1 | Commercial | Lipase B from *C. antarctica* (Lypozyme® CAL-B lipase, STREM chemicals) |
| Enzyme 2 | Commercial | Esterase from porcine liver (E3019, SIGMA-ALDRICH (Merck) |
| Enzyme 3 | Commercial | Cholesterol esterase from porcine pancreas (26745, SIGMA-ALDRICH (Merck)) |
| Enzyme 4 | Commercial | Lipase from *A. oryzae* (Novozym® 51032, STREM chemicals) |
| Enzyme 5 | In-house | Lipase<br>The amino sequence of which is SEQ ID NO: 4 |
| Enzyme 6 | In-house | BTA-hydrolase 1 (Cutinase)<br>The amino sequence of which is SEQ ID NO: 5 |
| Enzyme 7 | In-house | Carboxylesterase/ lipase family protein<br>The amino sequence of which is SEQ ID NO: 6 |
| Enzyme 8 | In-house | Putative lactonizing lipase<br>The amino sequence of which is SEQ ID NO: 7 |
| Enzyme 9 | In-house | Hydrolase of alpha/beta superfamily<br>The amino sequence of which is SEQ ID NO: 8 |

***Cloning, over-expression and purification of the 'in-house' enzymes of the invention***

Primers were chosen, genes were cloned and protein overexpressed in *E. coli* as previously described (Vergne-Vaxelaire et al., Adv. Synth. Catal. 2013, 355, 1763 - 1779). Each expression plasmid was transformed into *E. coli* (for example E. *coli* BL21-CodonPlus (DE3)-RIPL).

Cell culture, isopropyl b-D-thiogalactopyranoside (IPTG) induction of protein production and cell lysis were conducted as previously published (C. Guérard-Hélaine et al. ChemCatChem 2015, 7, 1871-1879).

The enzymes were purified by loading the cell-free extract onto a Ni-NTA column (QIAGEN) according to the manufacturer's instructions. The elution buffer was 50 mM phosphate (pH 7.5), 50 mM NaCl, 250 mM imidazole and 10% glycerol and the desalting buffer was 50 mM phosphate (pH 7.5), 50 mM NaCl and 10% glycerol.

**Results: Degrading activity of the enzymes of the invention towards CR-39® polymer cubes**

The enzymes that were selected with the BTB assay on CR-39® monomer (commercial, crude lysates and purified proteins) and listed in Table 1 above were tested for their capacity to degrade CR-39® polymer cubes (Hou *et al.,* 2019). Several experiments were conducted in which a cube of CR-39® polymer was mixed with one of the selected

enzymes in a solution of sodium phosphate buffer 50 mM, pH7. Two enzyme concentrations were tested, either 200 or 2000 mU. The reaction mixture was incubated at 50°C, under constant agitation (150 rpm). Every 7 days, the reaction mixture was removed, and fresh buffer and enzymes were added. The reaction was stopped after 35 days.

The degrading activity of each enzyme was determined by calculating the weight loss of the CR-39® polymer cube at the end of the reaction. The weight loss was calculated with the following formula:

$$\%\ \text{Degradation}\ (\%\ \text{weight loss}) = \frac{\text{Weight of polymer cube before reaction} - \text{Weight of polymer cube after reaction}}{\text{Weight of polymer cube before reaction}} \times 100$$

The results are shown in Figure 1.

Conclusions: Enzyme 1, enzyme 3, enzyme 6 in the form of crude lysate, enzyme 8 in the form of crude lysate and purified protein and enzyme 7 in the form of purified protein were observed to be active towards degradation of CR-39® polymer cubes with % degradation of 0.75%, 1.54%, 0.77%, 1.21%, 2.0%, and 0.76% respectively.

## REFERENCES

S. Tsushima, Y. Matsushita, Technical Report on the PCR-DGGE Analysis of Bacterial and Fungal Soil Communities, National Institute for Agro-Environmental Sciences, Tsukuba (2010).

A. Amobonye, P. Bhagwat, S. Singh, S. Pillai, Plastic biodegradation: frontline microbes and their enzymes, Sci. Total Environ., 759 (2021), p. 143536.

A.K. Urbanek, A.M. Mirończuk, A. Garcia-Martin, A. Saborido, I. Mata, M. Arroyo, Biochemical properties and biotechnological applications of microbial enzymes involved in the degradation of polyester-type plastics, BBA - Proteins and Proteomics, 1868 (2020), p. 140315.

K. Mukai, Y. Doi, Y. Sema, K. Tomita, Substrate specificities in hydrolysis of polyhydroxyalkanoates by microbial esterases, Biotechnol. Lett., 15 (1993), pp. 601-604.

Toru Shimada and Takeshi Hasegawa, Determination of equilibrium structures of bromothymol blue revealed by using quantum chemistry with an aid of multivariate analysis of electronic absorption spectra, Spectrochimica Acta Part A: Mol. Biomol. Spectr., 185, 2017, 104-110.

Nazim Ocal, Aurélie Lagarde, Mélanie L'enfant, Franck Charmantray, and Laurence Hecquet, High-Throughput Solid-Phase Assay for Substrate Profiling and Directed Evolution of Transketolase, ChemBioChem, 2021, 22, 1-8.

Zhipeng Hou, Wei Zhang, Jing Guob, Zhangpei Chena, Jianshe Hua, Liqun Yang, The in vitro enzymatic degradation of poly(trimethylene carbonate-co-2, 2'-dimethyltrimethylene carbonate), Eur. Polym. J., 2019, 112, 51-59.

Vergne-Vaxelaire et al., Nitrilase Activity Screening on Structurally Diverse Substrates: Providing Biocatalytic Tools for Organic Synthesis, Adv. Synth. Catal. 2013, 355, 1763-1779.

Christine Guérard-Hélaine et al., Genome Mining for Innovative Biocatalysts: New Dihydroxyacetone Aldolases for the Chemist's Toolbox, ChemCatChem, 2015, 7, 1871-1879.


## Claims

**1.** A method for the enzymatic degradation of poly(allyl diglycol carbonate), comprising contacting said polymer with at least one enzyme capable of degrading poly(allyl diglycol carbonate) and selected from the group consisting of :

- enzyme 1, the amino acid sequence of which presents at least 60% identity with the following amino acid sequence:

MKLLSLTGVAGVLATCVAATPLVKRLPSGSDPAFSQPKSVL DAGLTCQGASPSSVSKPILLVPGTGTTGPQSFDSNWIPLSTQLGYTPCWISP PPFMLNDTQVNTEYMVNAITALYAGSGNNKLPVLTWSQGGLVAQWGLTFFP SIRSKVDRLMAFAPDYKGTVLAGPLDALAVSAPSVWQQTTGSALTTALRNAG GLTQIVPTTNLYSATDEIVQPQVSNSPLDSSYLFNGKNVQAQAVCGPLFVIDH AGSLTSQFSYVVGRSALRSTTGQARSADYGITDCNPLPANDLTPEQKVAAAA LLAPAAAAIVAGPKQNCEPDLMPYARPFAVGKRTCSGIVTP (SEQ ID NO: 1 in the annexed sequence listing),

- enzyme 2, the amino acid sequence of which presents at least 60% identity with the following amino acid sequence:

MWLLPLVLTSLASSATWAGQPASPPVVDTAQGRVLGKYVS LEGLAQPVAVFLGVPFAKPPLGSLRFAPPQPAEPWSFVKNTTSYPPMCCQD PVVEQMTSDLFTNGKERLTLEFSEDCLYLNIYTPADLTKRGRLPVMVWIHGG GLVLGGAPMYDGVVLAAHENVVVVAIQYRLGIWGFFSTGDEHSRGNWGHL DQVAALHWVQENIANFGGDPGSVTIFGESAGGESVSVLVLSPLAKNLFHAIS ESGVALTVALVRKDMKAAAKQIAVLAGCKTTTSAVFVHCLRQKSEDELLDTL KMKFLTLDFHGDQRESHPFLPTVVDGVLLPKMPEEILAEKDFNTVPYIVGINK QEFGWLLPTMMGFPLSEGKLDQKTATSLLWKSYPIANIPEELTPVATDKYLG GTDDPVKKKDLFLDLMGDVVFGVPSVTVARQHRDAGAPTYMYEFQYRPSF SSDKKPKTVIGDHGDEIFSVFGFPLLKGDAPEEEVSLSKTVMKFWANFARSG NPNGEGLPHWPMYDQEEGYLQIGVNTQAAKRLKGEEVAFWNDLLSKEAAK KPPKIKHAEL (SEQ ID NO: 2 in the annexed sequence listing),

- enzyme 3, the amino acid sequence of which presents at least 60% identity with the following amino acid sequence:

MDLRTMTQSLVTLAEDNMAFFSGQGPGETARRLSGVFAGI REQALGLEPALGRLLSVAHLFDLDAETPANGYRSLVHTARCCLAHLLHKSRY VASNRRSIFFRTSHNLAELEAYLAALTQLRALAYYAQRLLAINRPGKLFFEGD EGITADFLREYVTLHKGCFYGRCLGFQFTPAIRPFLQTISIGLVSFGEHYKRNE TGLSVTASSLFTSGRFAIDPELRGAEFERIIQNLDVHFWKAFWNITEIEVLSSL ANMASATVRVSRLLSLPPKAFEMPLTADPKLTVTISPPLAHTGPGPVLVRLIS

YDLREGQDSEELSSLVRSEGPRGLELRPRPQQAPRSRSLVVHIHGGGFVAQ TSKSHEPYLKSWAQELGVPILSIDYSLAPEAPFPRALEECFYAYCWAVKHCG LLGSTGERICLAGDSAGGNLCFTVSLRAAAYGVRVPDGIMAAYPATMLQSAA SPSRLLSLMDPLLPLSVLSKCVSAYAGGEMEDHSDSDQKALGMMGLVRRDT ALLFRDLRLGASSWLNSFLELSGHKSRPNLVPTEEPMRRSVSEAALAQPEG PLGTDSLKYLTLHDLSLSSETQDTPELSLSAETLGPTTPSAVNFLFRPEDAPE EAEARDDISTKEEKVYSVRAAFPEGFHPRRSSQGAIQMPLYSAPIVKNPFMS PLLAPDSMLQTLPPVHIVACALDPMLDDSVMFARRLRSLGQPVTLHVVEDLP HGFLSLAALCRETRQAAALCVDRIRFILNPPGPATPAGPTTPPV (SEQ ID NO: 3 in the annexed sequence listing),

- enzyme 4 corresponding to a lipase from *Aspergillus oryzae,*
- enzyme 5, the amino acid sequence of which presents at least 60% identity with the following amino acid sequence:

MRKFTLGCLSATALVALSVSATAAPLPDTPGAPFPSVSSFD NDGPYAVTSQSEGPNCRVYRPRTLGQGGVRHPIILWGNGTGTGPTTYSGLL THWASHGFVVAAAETSNAGTGREMLACLDYLVQESNRTYGTYVGVLNTGR VGTSGHSQGGGGSIMAGQDDRVKATAPIQPYTIGLGHDSSSQRNQRGPMF LMSGGADTIAIPYLNAQPVFTRANVPIFWGERRYVSHFEPVGNGGEYRGPST AWFRYQLMDDQSARSTFYGRLCRLCTSLLWSVERKGIE (SEQ ID NO: 4 in the annexed sequence listing),

- enzyme 6, the amino acid sequence of which presents at least 60% identity with the following amino acid sequence:

MAVMTPRRERSSLLSRALQVTAAAATALVTAVSLAAPAHAA NPYERGPNPTDALLEASSGPFSVSEENVSRLSASGFGGGTIYYPRENNTYG AVAISPGYTGTEASIAWLGERIASHGFVVITIDTITTLDQPDSRAEQLNAALNH MINRASSTVRSRIDSSRLAVMGHSMGGGGTLRLASQRPDLKAAIPLTPWHLN KNWSSVTVPTLIIGADLDTIAPVATHAKPFYNSLPSSISKAYLELDGATHFAPNI PNKIIGKYSVAWLKRFVDNDTRYTQFLCPGPRDGLFGEVEEYRSTCPF (SEQ ID NO: 5 in the annexed sequence listing),

- enzyme 7, the amino acid sequence of which presents at least 60% identity with the following amino acid sequence:

MTELTVQTRCGALKGTAGHGVRTWKSIPYAKPPVGELRFKA PEPPVPWDGVKNADSFGPVCPQPADLLSMSFSGDVPPQSEDCLYLNVFAP DSEGGKRPVMVWIHGGAFFLGAGSEPTYDASALAADGDVIVVTLNYRLGPF GFLHLFSIDDTYPGNIGMLDQIAALRWVKDNISAFGGDPDNVTVFGESAGGM SIASLMAMPDAKGLFHKAILESGASQTMTADVAKEITTAFIQEAGTDQLQELS VNDILKTADKLRNTIDQSIFHLLFQPAIDPATLPAEPAKAIADGAAEGIPMIIGTN RDEAYLFFTPDTDIHSEKKQQEYLLYHLGENSAKLAADLYPHSLTGQIDMMT

DLKFWRPAVAFAQEQSQYAPVWMYRFDWHGETPPFHKAVHALELPFVFGN FDSLKKTLKEPLSEDVKQLSKLIQSAWIAFAKTGKPDTDQLHWPQYETGSRE TIIFNTSVSTESDPDSAKRRILFQA (SEQ ID NO: 6 in the annexed sequence listing),

- enzyme 8, the amino acid sequence of which presents at least 60% identity with the following amino acid sequence:

MSASSLKFPIVLVHGLLGFDKIGGIYPYFYGIKEALEKAGAKV YIATLSALNSNELRGEQLLEFVRKVQAETGAAKVNLIGHSQGPLACRYVAAT HPELIASVTSVNGVNHGSEVADLVRLALTPGRLPESIANAAMSAFGQLLSALA GSPRLPQSGIEALEALTSEGVAAFNKKYPQGLPAEWGGEGKELVNGVYYYS WSGIIDYNPLHQGANNLDPLHVAMLAFSILFTNERFQNDGLVGRYSSHLGKVI GSDYSMDHVDAINQLAGVVANNTDPVQLFVEHVARLKSKGL (SEQ ID NO: 7 in the annexed sequence listing),

and

- enzyme 9, the amino acid sequence of which presents at least 60% identity with the following amino acid sequence:

MKKLVALLIVGLFVSISLTGLGLAGGTAPYKCDTRYPVVLAH GMGASTKILGIVDYWYGIEDALKAEGASVYFTSVNAMGSTVDKAADFKQQF MEILAVTGAPKANIIGHSHGTLYTRYAISNLGLAPYVASYTSLAGPHRGSAVA SLIMYDLPDWLLAAGGDVLNFVYTFIFGDTNPDSLQNALDLCPDYMVNTFNP NTPNIPGIYYQSWAAKAKTSCPSVILEPTWLIMLIEEGANDGLVSVESAKWGN FRGVEDAAWYSAGCDHLNIVGQLFGVTPGFDAPQFFVDIVEDLKGRGY (SEQ ID NO: 8 in the annexed sequence listing).

2. The method for the enzymatic degradation of poly(allyl diglycol carbonate) according to claim 1, wherein said enzyme 1 is a lipase B from *Candida antarctica*.

3. The method for the enzymatic degradation of poly(allyl diglycol carbonate) according to claim 1 or 2, wherein said enzyme 1 is the commercial lipase Lipozyme® CAL-B.

4. The method for the enzymatic degradation of poly(allyl diglycol carbonate) according to any one of claims 1 to 3, wherein said enzyme 2 is an esterase from porcine liver.

**5.** The method for the enzymatic degradation of poly(allyl diglycol carbonate) according to any one of claims 1 to 4, wherein said enzyme 2 is a commercial esterase from porcine liver.

**6.** The method for the enzymatic degradation of poly(allyl diglycol carbonate) according to any one of claims 1 to 5, wherein said enzyme 3 is a cholesterol esterase from porcine pancreas.

**7.** The method for the enzymatic degradation of poly(allyl diglycol carbonate) according to any one of claims 1 to 6, wherein said enzyme 3 is a commercial cholesterol esterase from porcine pancreas.

**8.** The method for the enzymatic degradation of poly(allyl diglycol carbonate) according to any one of claims 1 to 7, wherein said enzyme 4 is the commercial lipase Novozym® 51032 from *Aspergillus oryzae.*

**9.** The method for the enzymatic degradation of poly(allyl diglycol carbonate) according to any one of claims 1 to 8, wherein said at least one enzyme is used in the form of a pure protein, a crude lysate or immobilized on a support.

**10.** Use of the method according to any one of claims 1 to 9, for producing fragments, oligomers, monomers or repeating units derivatives of poly(allyl diglycol carbonate) that can be recovered and recycled for obtaining poly(allyl diglycol carbonate).

**11.** A kit for the enzymatic degradation of poly(allyl diglycol carbonate), comprising at least one enzyme as defined in any one of claims 1 to 8.

**12.** The kit of claim 11, further comprising at least one reagent for the degradation reaction.

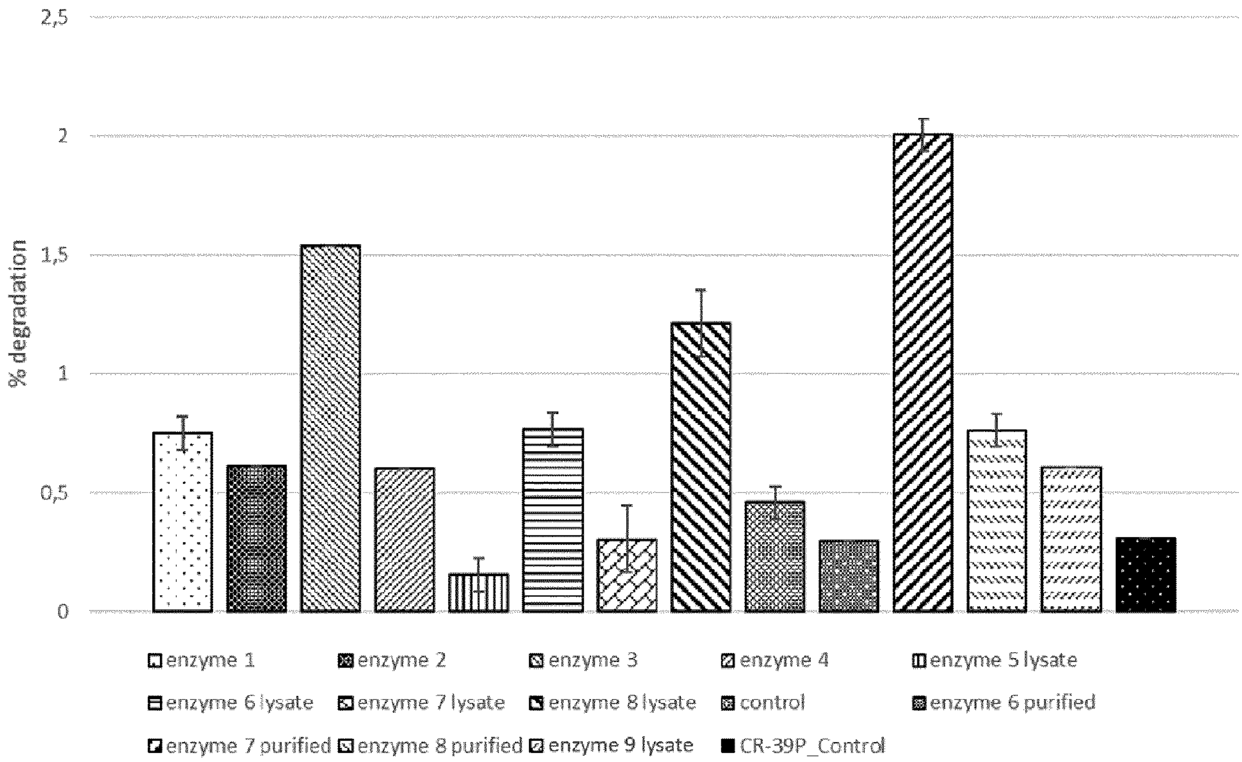
2,5
2
1,5
1
0,5
0
% degradation
enzyme 1
enzyme 2
enzyme 3
enzyme 4
enzyme 5 lysate
enzyme 6 lysate
enzyme 7 lysate
enzyme 8 lysate
control
enzyme 6 purified
enzyme 7 purified
enzyme 8 purified
enzyme 9 lysate
CR-39P_Control

Fig. 1

Europäisches Patentamt
European Patent Office
Office européen des brevets

# EUROPEAN SEARCH REPORT

Application Number
EP 23 30 6473

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/278297 A1 (DANISCO US INC [US]) 5 January 2023 (2023-01-05) * [006],[0011],[0015]-[0016] * -& DATABASE GSP [Online] 5 January 2023 (2023-01-05), Adams C. ET AL: "Pseudomonas mendocina wild-type lipase, SEQ 2 of WO2023278297-A1", XP093115223, Database accession no. BMH27349 * abstract * ----- | 1,9,10 | INV. C12P7/04 C12N9/18 C12N9/24 C12P7/26 C12P7/62 |
| A | FRIEDMANN G ET AL: "Thermal degradation of poly(diethyleneglycol-bis-allyl carbonate)", POLYMER DEGRADATION AND STABILITY, BARKING, GB, vol. 4, no. 3, 1 May 1982 (1982-05-01), pages 195-202, XP024143769, ISSN: 0141-3910, DOI: 10.1016/0141-3910(82)90026-X [retrieved on 1982-05-01] * abstract; figure 2; table 1 * ----- | 1-10 | TECHNICAL FIELDS SEARCHED (IPC) C12P C12N |
| A | CN 116 535 545 A (SHANXI INST COAL CHEMISTRY CAS) 4 August 2023 (2023-08-04) * the whole document * ----- | 1-10 | |
| | -/-- | | |

The present search report has been drawn up for all claims

2

| Place of search | Date of completion of the search | Examiner |
|---|---|---|
| Berlin | 2 January 2024 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 30 6473

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Novozymes Lipase Screening Kit purchased from Strem Chemicals Strem Product Catalogue", , 1 January 2023 (2023-01-01), pages 1-2, XP093115221, Retrieved from the Internet: URL:https://www.strem.com/catalog/v/96-0220/novozymes_lipase_screening_kit_contains_9_lipase_enzymes * the whole document * | 11,12 | |
| X | -& Syldatk Christoph ET AL: "Lipase-catalysed synthesis of glycolipids -utilization of innovative media, reactors and substrates", , 17 August 2021 (2021-08-17), pages 1-134, XP093115222, Retrieved from the Internet: URL:https://publikationen.bibliothek.kit.edu/1000136597 [retrieved on 2023-12-31] * item 2.2.1 * ----- | 11,12 | TECHNICAL FIELDS SEARCHED (IPC) |

The present search report has been drawn up for all claims

2

| Place of search | Date of completion of the search | Examiner |
|---|---|---|
| Berlin | 2 January 2024 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 30 6473

This annex lists the patent family members relating to the patent documents cited in the above-mentioned European search report. The members are as contained in the European Patent Office EDP file on
The European Patent Office is in no way liable for these particulars which are merely given for the purpose of information.

02-01-2024

| Patent document cited in search report | Publication date | Patent family member(s) | Publication date |
|---|---|---|---|
| WO 2023278297 A1 | 05-01-2023 | NONE | |
| CN 116535545 A | 04-08-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 25656-90-0
- *CHEMICAL ABSTRACTS*, 9016-18-6
- *CHEMICAL ABSTRACTS*, 9026-00-0
- **VERGNE-VAXELAIRE et al.** *Adv. Synth. Catal*, 2013, vol. 355, 1763-1779
- **HÉLAINE et al.** *ChemCatChem*, 2015, vol. 7, 1871-1879
- **S. TSUSHIMA** ; **Y. MATSUSHITA**. Technical Report on the PCR-DGGE Analysis of Bacterial and Fungal Soil Communities. *National Institute for Agro-Environmental Sciences, Tsukuba*, 2010
- **A. AMOBONYE** ; **P. BHAGWAT** ; **S. SINGH** ; **S. PILLAI**. Plastic biodegradation: frontline microbes and their enzymes. *Sci. Total Environ.*, 2021, vol. 759, 143536
- **A.K. URBANEK** ; **A.M. MIROŃCZUK** ; **A. GARCIA-MARTIN** ; **A. SABORIDO** ; **I. MATA** ; **M. ARROYO**. Biochemical properties and biotechnological applications of microbial enzymes involved in the degradation of polyester-type plastics. *BBA - Proteins and Proteomics*, 2020, vol. 1868, 140315
- **K. MUKAI** ; **Y. DOI** ; **Y. SEMA** ; **K. TOMITA**. Substrate specificities in hydrolysis of polyhydroxyalkanoates by microbial esterases. *Biotechnol. Lett.*, 1993, vol. 15, 601-604
- **TORU SHIMADA** ; **TAKESHI HASEGAWA**. Determination of equilibrium structures of bromothymol blue revealed by using quantum chemistry with an aid of multivariate analysis of electronic absorption spectra. *Spectrochimica Acta Part A: Mol. Biomol. Spectr.*, 2017, vol. 185, 104-110
- **NAZIM OCAL** ; **AURÉLIE LAGARDE** ; **MÉLANIE L'ENFANT** ; **FRANCK CHARMANTRAY** ; **LAURENCE HECQUET**. High-Throughput Solid-Phase Assay for Substrate Profiling and Directed Evolution of Transketolase. *ChemBioChem*, 2021, vol. 22, 1-8
- **ZHIPENG HOU** ; **WEI ZHANG** ; **JING GUOB** ; **ZHANGPEI CHENA** ; **JIANSHE HUA** ; **LIQUN YANG**. The in vitro enzymatic degradation of poly(trimethylene carbonate-co-2, 2'-dimethyltrimethylene carbonate). *Eur. Polym. J*, 2019, vol. 112, 51-59
- **VERGNE-VAXELAIRE et al.** Nitrilase Activity Screening on Structurally Diverse Substrates: Providing Biocatalytic Tools for Organic Synthesis. *Adv. Synth. Catal*, 2013, vol. 355, 1763-1779
- **CHRISTINE GUÉRARD-HÉLAINE et al.** Genome Mining for Innovative Biocatalysts: New Dihydroxyacetone Aldolases for the Chemist's Toolbox. *ChemCatChem*, 2015, vol. 7, 1871-1879